# EUROPEAN PATENT APPLICATION

(11) **EP 4 736 772 A1**
(43) Date of publication of application: **06.05.2026**
(21) Application number: 25212434.2
(22) Date of filing: 30.10.2025
(51) Int. Cl.: A61B 6/40, A61B 6/03, A61B 6/04, A61B 6/00, A61B 6/58, A61N 5/10

(54) **IMAGING SYSTEM AND METHOD, RADIOTHERAPY SYSTEM, AND ELECTRONIC DEVICE**

(30) Priority: 01.11.2024 CN 202411555651
(71) Applicant: Our United Corporation, Xi'an City, Shaanxi 710018 (CN)
(72) Inventor: LIU, Haifeng, Xi'an City, Shaanxi, 710018 (CN); ZHANG, Zhongyuan, Xi'an City, Shaanxi, 710018 (CN); LI, Jiuliang, Xi'an City, Shaanxi, 710018 (CN); WANG, Zhongya, Xi'an City, Shaanxi, 710018 (CN); ZHANG, Shenglin, Xi'an City, Shaanxi, 710018 (CN); XU, Qiuhao, Xi'an City, Shaanxi, 710018 (CN)
(74) Representative: Whiting, Gary

(57) **Abstract**

Provided are an imaging system and method, a radiotherapy system, and an electronic device, belonging to the medical field of technologies. The imaging system of the present disclosure is configured to image a target object (1), and includes: a radiation source (2), configured to emit a beam (201) to the target object (1); a detector (3), arranged opposite to the radiation source (2); and a diagnosis and treatment couch (5), disposed between the radiation source (2) and the detector (3), fixed relative to the detector (3), and configured to bear and drive the target object (1) to move. During movement of the diagnosis and treatment couch (5), the beam (201) emitted by the radiation source (2) always partially or entirely overlaps with a target volume of the target object (1), and the beam (201) passing through the target volume of the target object (1) is receivable by the detector (3).

## Description

### TECHNICAL FIELD

The present disclosure relates to the medical field, in particular to the field of target volume imaging technologies, and particularly to an imaging system and method, a radiotherapy system, and an electronic device.

### BACKGROUND OF THE INVENTION

With the rapid development of computer and imaging technologies, image-guided therapy technologies are playing an increasingly important role in improving the accuracy of radiotherapy. These technologies improve the local control rate of tumors and reduce the irradiation dose to surrounding normal tissues, which in turn reduces damage to normal tissues.

### SUMMARY OF THE INVENTION

The present disclosure provides an imaging system and method, a radiotherapy system, and an electronic device.

In a first aspect, the present disclosure provides an imaging system. The imaging system is configured to image a target object, and the imaging system includes:
a radiation source, configured to emit a beam to the target object;
a detector, arranged opposite to the radiation source; and
a diagnosis and treatment couch, disposed between the radiation source and the detector, fixed relative to the detector, and configured to bear and drive the target object to move;
wherein during movement of the diagnosis and treatment couch, the beam emitted by the radiation source always partially or entirely overlaps with a target volume of the target object, and the beam passing through the target volume of the target object is receivable by the detector.

In some embodiments, a plurality of radiation sources are provided.

In some embodiments, central axes of the beams of the plurality of radiation sources intersect at an imaging point.

In some embodiments, angles between central axes of the beams of the plurality of radiation sources, and mounting positions, arrangement directions, and an excitation sequence of the plurality of radiation sources are all adjustable.

In some embodiments, the plurality of radiation sources are arranged in a cranio-caudal direction of the target object.

In some embodiments, a straight-line distance between the detector and the radiation source is adjustable.

In some embodiments, the diagnosis and treatment couch includes a couch board and a couch base, the couch board being fixed on the couch base, wherein a position of the couch board is fixed relative to a position of the detector.

In some embodiments, the detector is disposed on the couch board.

In some embodiments, the couch board and the detector are capable of reciprocating separately along an X axis, a Y axis, and a Z axis in an IEC coordinate system.

In some embodiments, the imaging system further includes: an imaging control processing device, capable of controlling, based on an imaging mode selected by a user, the imaging system as described above to perform imaging.

In some embodiments, the imaging mode includes tomography, KV imaging, or CBCT imaging.

In some embodiments, the size of the detector is selectable according to actual situations. In some embodiments, the detector is sized to be able to cover a part, where the target volume is located, of the target object.

In a second aspect, the present disclosure provides a radiotherapy system, including: the imaging system as described above; and a radiotherapy device.

In some embodiments, an overlapping volume between the beam emitted by the radiation source and the target volume of the target object always partially or entirely overlaps with a treatment volume of the radiotherapy device.

In some embodiments, during movement of the diagnosis and treatment couch from a positioning reference point to a radiotherapy isocenter of the radiotherapy device, the imaging system monitors a position of the target volume of the target object, and an imaging point of the imaging system moves along a connecting line between the positioning reference point and the radiotherapy isocenter;
wherein the connecting line between the positioning reference point and the radiotherapy isocenter is parallel to an X axis, a Y axis, or a Z axis in an IEC coordinate system of the radiotherapy device.

In a third aspect, the present disclosure provides an imaging method, applicable to the imaging system as described above, and the method including: adjusting the radiation source, to enable that, during movement of the diagnosis and treatment couch, the beam emitted by the radiation source always partially or entirely overlaps with a target volume of the target object, and the beam passing through the target volume of the target object is receivable by the detector; and controlling the detector to generate image data.

In some embodiments, a plurality of radiation sources are provided, and adjusting the radiation source includes at least one of:
adjusting a number of the plurality of radiation sources;
adjusting mounting positions of the plurality of radiation sources;
adjusting arrangement directions of the plurality of radiation sources;
adjusting an excitation sequence of the plurality of radiation sources; or
adjusting angles between central axes of beams of the plurality of radiation sources.

In a fourth aspect, the present disclosure provides an electronic device. The electronic device includes: a processor; and a memory, configured to store one or more instructions executable by the processor;
wherein the processor, when executing the one or more instructions, is caused to perform the imaging method as described above.

### BRIEF DESCRIPTION OF DRAWINGS

The accompanying drawings are provided for better understanding of the solutions and do not constitute a limitation to the present disclosure, in which:
FIG. 1 is a schematic diagram of a scenario of an imaging system according to some embodiments of the present disclosure;
FIG. 2 is a schematic diagram of a scenario of a radiotherapy system according to some embodiments of the present disclosure;
FIG. 3 is a schematic structural diagram of a radiotherapy system according to some embodiments of the present disclosure;
FIG. 4 is a schematic diagram of a scenario of real-time imaging according to some embodiments of the present disclosure;
FIG. 5 is a schematic block diagram of an electronic device according to some embodiments of the present disclosure; and
FIG. 6 is a flowchart of an imaging method according to some embodiments of the present disclosure.

### DETAILED DESCRIPTION

The technical solutions in the embodiments of the present disclosure are clearly and entirely described hereinafter with reference to the accompanying drawings in the embodiments of the present disclosure. Apparently, the described embodiments are only some but not all of the embodiments of the present disclosure. All other embodiments acquired by those of ordinary skill in the art without creative efforts based on the embodiments in the present disclosure are within the protection scope of the present disclosure.

The term "and/or" herein merely describes an associative relationship between related objects, indicating three possible relationships. For example, "A and/or B" includes cases: A exists alone, A and B exist concurrently, and B exists alone.

In the descriptions of the present disclosure, it should be understood that orientation or positional relationships indicated by terms "center," "length," "width," "upper," "lower," etc. are orientation or positional relationships shown based on the drawings, these terms are used only for ease of describing the present disclosure and simplifying the descriptions, but not indicating or implying that the specified apparatus or element has to be specifically disposed, or structured and operated in a specific direction, and therefore, should not be understood as limitations to the present disclosure. In the descriptions of the present disclosure, the term "plurality" means two or more, unless otherwise expressly and specifically defined.

In the descriptions of the present disclosure, the word "exemplary" is used to indicate "examples, illustrations, or explanations". Any embodiment described as "exemplary" in the descriptions of the present disclosure is not necessarily to be construed as being more preferable or advantageous than other embodiments. The following descriptions are given to enable any person skilled in the art to implement and adopt the present disclosure. In the following descriptions, details are set forth for the purpose of explanation. It should be appreciated that those of ordinary skills in the art can recognize that the present disclosure can be implemented without using these particular details. In other examples, well-known structures or processes will not be explained in detail to avoid obscuring the descriptions of the present disclosure with unnecessary details. Therefore, the present disclosure is not intended to be limited to the illustrated embodiments, but is consistent with the broadest scope that conforms to the principles and features disclosed in the present disclosure.

It should be noted that the method according to the embodiments of the present disclosure is implemented in an imaging control processing device, and various processing objects of the imaging control processing device all exist in the form of data or information. For example, time is essentially time information. It can be understood that the size, quantity, position, etc. mentioned in the subsequent embodiments should all exist in the form of corresponding data to facilitate processing by the imaging control processing device, the details of which will not be repeated herein.

With the rapid development of computer and imaging technologies, image-guided therapy technologies are playing an increasingly important role in improving the accuracy of radiotherapy. These technologies improve the local control rate of tumors and reduce the irradiation dose to surrounding normal tissues, which in turn reduces damage to normal tissues.

In order to avoid a locating error caused by organ movements (such as breathing), a change in the position of a target object and other factors, and to accurately locate a tumor tissue, a real-time or near-real-time imaging technology is employed to locate a tumor in the process of treatment, thereby reducing damage to healthy tissues and improving the treatment effect.

In the related art, image-guided imaging technologies mainly include cone-beam CT imaging, dual-flat-panel orthogonal imaging, and magnetic resonance imaging, in which the dual-flat-panel orthogonal imaging is quasi-three-dimensional imaging with low imaging dose and high imaging speed. However, an existing dual-flat-panel orthogonal system requires two detectors respectively disposed on two sides of a target object and two radiation sources arranged respectively opposite to the two detectors, which increases the complexity of devices and occupies much space. In terms of angle, the existing dual-flat-panel orthogonal imaging system has a fixed angle, which is not only limited in imaging angle and thus makes it difficult to acquire multi-view information in a complex anatomical structure, but also limited in the field of view, thus results in the inability to entirely observe lesions or anatomical regions within a larger range and a high possibility of missing important peripheral information, thereby affecting the accuracy of diagnosis. In addition, the existing dual-flat-panel orthogonal imaging system has a non-adjustable angle; thus, it does not have the function of multi-angle real-time imaging and cannot meet some rapidly changing diagnostic requirements. Moreover, in the case that the existing dual-flat-panel orthogonal imaging system is used for imaging, the target object needs to maintain a specific posture accurately, which leads to high requirements on the posture of the target object.

In view of the above technical problems, the present disclosure provides an imaging system. The imaging system according to the present disclosure reduces the complexity of devices, is capable of quickly and accurately acquiring an image of a target volume of the target object in the moving process, and is capable of achieving real-time multi-angle monitoring of the position of the target volume of the target object.

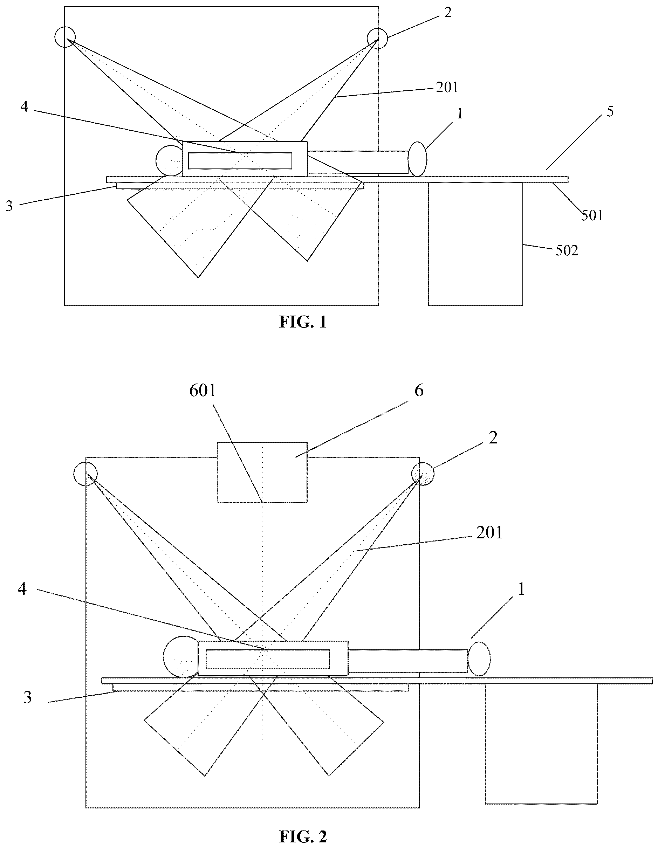

FIG. 1 is a schematic diagram of a scenario of an imaging system according to some embodiments of the present disclosure. The imaging system according to the present disclosure is configured to image a target object 1. Referring to FIG. 1, the imaging system according to the present disclosure includes: a radiation source 2, configured to emit a beam 201 to the target object 1; a detector 3, arranged opposite to the radiation source 2; and a diagnosis and treatment couch 5, disposed between the radiation source 2 and the detector 3, fixed relative to the detector 3, and configured to bear and drive the target object 1 to move. During movement of the diagnosis and treatment couch 5, the beam 201 emitted by the radiation source 2 always partially or entirely overlaps with a target volume of the target object 1, and the beam 201 passing through the target volume of the target object 1 is receivable by the detector 3. In the present disclosure, the beam 201 emitted by the radiation source 2 always partially or entirely overlapping with the target volume of the target object 1 means that the beam 201, when reaching the position where the target volume of the target object 1 is located, entirely or at least partially overlaps with the target volume of the target object 1.

It should be noted that the moving process of the diagnosis and treatment couch 5 refers to a moving process of the diagnosis and treatment couch 5 from a positioning reference point to a radiotherapy isocenter.

It should be noted that by making the positions of the diagnosis and treatment couch 5 and the detector 3 fixed relative to each other, the complexity of devices is reduced, the imaging operation is simplified, and the imaging time is shortened, such that an image of the target volume of the target object in the moving process is acquired quickly and accurately, and real-time multi-angle monitoring on the position of the target volume of the target object 1 is achieved.

The manner in which the diagnosis and treatment couch 5 and the detector 3 move is not limited in the present disclosure, as long as the positions of the diagnosis and treatment couch 5 and the detector 3 are fixed relative to each other. In some embodiments, the diagnosis and treatment couch 5 and the detector 3 move simultaneously. In some embodiments, the diagnosis and treatment couch 5 and the detector 3 move independently.

In some embodiments, a ray emitted by the radiation source 2 is the beam 201 (such as an X-ray), and the beam 201 passes through the target volume of the target object 1 and then is received by the detector 3.

In some embodiments, the radiation source 2 is a conventional apparatus (such as a bulb tube) in the art capable of emitting the beam 201.

In some embodiments, a plurality of radiation sources 2 are provided. In some embodiments, the number of the radiation sources 2 is two.

In some embodiments, the number of the radiation sources 2 is two or more, and central axes of the beams 201 of the two or more radiation sources 2 intersect at an imaging point 4.

In some embodiments, the imaging point 4 coincides with a central point of the target volume of the target object 1.

In some embodiments, a plurality of radiation sources 2 are provided, and angles between central axes of the beams 201 of the plurality of radiation sources, and mounting positions, arrangement directions, and an excitation sequence of the plurality of radiation sources 2 are all adjustable. The aforementioned adjustment is intended to ensure that during movement of the diagnosis and treatment couch 5, the beams 201 emitted by the radiation sources 2 always partially or entirely overlap with the target volume of the target object 1, and the beams 201 passing through the target volume of the target object 1 are receivable by the detector 3.

In some embodiments, the imaging system includes an adjusting apparatus, wherein the adjusting apparatus is configured to adjust one or more of a beam-exiting angle, a mounting position, and an arrangement direction of the radiation source 2. In some embodiments, the adjusting apparatus adjusts the angles between the central axes of the beams 201 of the different radiation sources 2 by adjusting the beam-exiting angles of the different radiation sources 2.

In some embodiments, the radiation sources 2 are distributed in a cranio-caudal direction or a direction connecting two arms of the target object 1. The cranio-caudal direction of the target object refers to a direction of a connecting line between a head to a foot in the case that the target object is positioned to a target posture; and the direction connecting two arms of the target object refers to a direction of a connecting line between arms on two sides in the case that the target object is positioned the target posture. In some embodiments, the target posture herein is a posture required for subsequent imaging or radiotherapy.

In some embodiments, the radiation sources 2 are arranged in a cranio-caudal direction of the target object 1. In this embodiment, the arrangement of the radiation sources 2 in the cranio-caudal direction of the target object 1 effectively reduces the imaging blind volume, improves the imaging comprehensiveness, and reduces the requirements on the posture of the target object.

In some embodiments, the imaging system includes a plurality of radiation sources 2 and a detector 3 disposed opposite to the plurality of radiation sources 2. In this embodiment, rays of the plurality of radiation sources 2 are received by the same detector 3.

In some embodiments, the number of the radiation sources 2 is two, and the two radiation sources 2 are arranged in a cranio-caudal direction of the target object 1.

Central axes of beams 201 of the two radiation sources 2 intersect at an imaging point 4.

In some embodiments, the imaging point 4 coincides with a central point of the target volume of the target object 1. The imaging is more accurate in the case that the imaging point 4 coincides with the central point of the target volume of the target object 1.

In some embodiments, a straight-line distance between the detector 3 and the radiation source 2 is adjustable. In some embodiments, the straight-line distance between the detector 3 and the radiation source 2 is adjusted to ensure that positions of the detector 3 and the diagnosis and treatment couch 5 relative to each other always remain fixed during movement of the diagnosis and treatment couch 5.

In some embodiments, a side surface of the detector 3 for receiving the beam 201 is flat or curved.

The size of the detector 3 is not limited in the embodiments of the present disclosure. In some embodiments, the size of the detector 3 is sufficient to cover a part, where the target volume is located, of the target object 1. That is, the size of the detector 3 ensures that the target volume of the target object 1 is completely imaged. In terms of a projection relationship, an orthographic projection of the part, where the target volume is located, of the target object 1 on a detection face of the detector entirely falls within a detection range of the detector.

In the present disclosure, the length of the detector 3 is selected according to actual situations. In some embodiments, the length of the detector 3 is 48 inches, i.e., 121.92 cm.

In the present disclosure, the width of the detector 3 is selected according to actual situations. In some embodiments, the width of the detector 3 is 17 inches, i.e., 43.18 cm.

In some embodiments, the detector 3 includes a Mercu 1748V flat panel detector or a Venu 1748V flat panel detector.

In the present disclosure, the pixel size of the detector 3 is selected according to actual situations. In some embodiments, the pixel size of the detector 3 is 139 µm.

In the present disclosure, the number of detectors 3 is selected according to actual situations. In some embodiments, the number of the detector 3 is one.

In some embodiments, the imaging system includes: two radiation sources 2, configured to emit beams 201 to the target object 1; one detector 3, arranged opposite to the radiation sources 2; and a diagnosis and treatment couch 5, disposed between the radiation sources 2 and the detector 3, fixed relative to the detector 3, and configured to bear and drive the target object 1 to move. During movement of the diagnosis and treatment couch 5, the beams 201 emitted by the radiation sources 2 always partially or entirely overlap with a target volume of the target object 1, and the beams 201 passing through the target volume of the target object 1 are receivable by the detector 3.

In the above embodiments, one detector 3 is used to replace an original dual-detector structure, which achieves the effects of reducing the complexity of devices, saving space, and reducing the difficulty of operation.

In the above embodiments, the two radiation sources 2 are arranged in the cranio-caudal direction of the target object 1. In the case that the radiation sources 2 are arranged in the cranio-caudal direction of the target object 1 and directed to the same detector 3, longitudinal image acquisition can be realized. Further, multi-angle two-dimensional image data are acquired by alternating excitation of the radiation sources 2 disposed at the head position and tail position. The term "longitudinal" herein refers to a length direction of the target object (a height direction of the target object when standing), a length direction of the diagnosis and treatment couch, or the cranio-caudal direction as described above. The term "tail" herein refers to the position of the foot of the target object.

In some embodiments, central axes of the beams 201 of the two radiation sources 2 intersect at an imaging point 4.

In some embodiments, the imaging point 4 overlaps with a central point of the target volume of the target object 1.

In some embodiments, the detector 3 includes a Mercu 1748V flat panel detector or a Venu 1748V flat panel detector.

In some embodiments, the diagnosis and treatment couch 5 includes a couch board 501 and a couch base 502, the couch board 501 being fixed on the couch base 502, wherein the position of the couch board 501 is fixed relative to the position of the detector 3.

In some embodiments, the detector 3 is disposed on the couch board 501. In some embodiments, the detector 3 is fixed to a surface of the couch board 501, or the detector 3 is embedded into the couch board 501.

The manner of fixing is not limited in the present disclosure. In some embodiments, the detector 3 is fixed by means of sticking.

In the above embodiments, the detector 3 is disposed on the couch board 501, which ensures that during movement of the diagnosis and treatment couch 5, positions of the detector 3 and the diagnosis and treatment couch 5 are always fixed relative to each other, and the detector 3 moves with the movement of the diagnosis and treatment couch 5.

In some embodiments, the couch board 501 and the detector 3 are capable of reciprocating separately along an X axis, a Y axis, and a Z axis in an IEC (International Electrotechnical Commission) coordinate system. In the IEC coordinate system, the X axis is parallel to a width direction of the diagnosis and treatment couch 5, the Y axis is parallel to a length direction of the diagnosis and treatment couch 5, and the Z axis is parallel to a height direction of the diagnosis and treatment couch 5.

In the present disclosure, the couch board 501 of the diagnosis and treatment couch 5 is capable of reciprocating along the X axis, the Y axis, and the Z axis, which ensures that the target volume of the target object 1 is able to be moved into an imaging region of the imaging system during imaging. The detector 3 is capable of reciprocating along the X axis, the Y axis, and the Z axis, which ensures that the positions of the detector 3 and the couch board 501 are fixed relative to each other.

In the above embodiments, angles between central axes of the beams 201 of the different radiation sources 2 of the imaging system, and mounting positions, arrangement directions, and an excitation sequence of the plurality of radiation sources 2 are all adjustable, to enable that during movement of the diagnosis and treatment couch 5, the beams 201 emitted by the radiation sources 2 always partially or entirely overlap with the target volume of the target object 1, and the beams 201 passing through the target volume of the target object 1 are receivable by the detector 3. In this way, the position of the part, where the target volume is located, of the target object is monitored in real time from multiple angles during movement of the diagnosis and treatment couch.

In some embodiments, the imaging system further includes: an imaging control processing device, capable of controlling, based on an imaging mode selected by a user, the imaging system described above to perform imaging.

In some embodiments, the imaging mode of the imaging system includes tomography, kilovoltage (KV) imaging, or cone-beam computed tomography (CBCT) imaging.

Tomography means to perform tomography on the target volume of the target object 1 in different directions.

CBCT imaging means to perform imaging in different directions on the part, where the target volume is located, of the target object 1.

In some embodiments, a computer system is running on the imaging control processing device, and the computer system is configured to perform an imaging method implemented by the imaging control processing device.

In some embodiments, the imaging control processing device is a computer device with a graphical user interface (GUI), and the computer device includes one or more processors, a memory, and one or more application programs. In some embodiments, the imaging control processing device includes an imaging system application program, and the processor of the imaging control processing device, when executing the imaging system application program, is caused to perform: reconstructing a plurality of sub-projection images in projection images corresponding to various groups of light sources with various focuses, so as to acquire a three-dimensional image of a target soft tissue.

In the embodiments of the present disclosure, the entity of the imaging control processing device is a terminal or a server, which is not limited in the embodiments of the present disclosure.

In some embodiments, the above terminal includes at least one of a smartphone, a smart watch, a desktop computer, a laptop computer, a virtual reality terminal, an augmented reality terminal, a wireless terminal, a portable laptop computer, or the like.

In some embodiments, the above server is a standalone physical server, or a server cluster or distributed file system constituted by a plurality of physical servers, or at least one of cloud servers that provide basic cloud computing services such as cloud service, cloud database, cloud computing, cloud function, cloud storage, network service, cloud communication, middleware service, domain name service, security service, content distribution network, big data, or artificial intelligence platform, etc., which is not limited in the embodiments of the present disclosure. In some embodiments, there are more or fewer servers, which is not limited in the embodiments of the present disclosure. Of course, in some embodiments, the server further includes other functions, so as to provide more comprehensive and diversified services.

In the imaging system provided by the present disclosure, the positions of the detector and the diagnosis and treatment couch are fixed relative to each other, so that the purposes of reducing the complexity of devices, saving space, and reducing the difficulty of operation are achieved. During movement of the diagnosis and treatment couch, the beam emitted by the radiation source always partially or entirely overlaps with the target volume of the target object, and the beam passing through the target volume of the target object is receivable by the detector, which avoids the problem that a detector of a dual-flat-panel orthogonal system in the related art needs to be frequently moved or adjusted in angle, and simplifies the operation of imaging and shortens the imaging time. Therefore, an image of the target volume of the target object in the moving process is acquired quickly and accurately, and the position of the target volume of the target object is monitored in real time from multiple angles.

On this basis, the radiation sources are arranged in the cranio-caudal direction of the target object in some embodiments, which effectively reduces the imaging blind volume, improves the imaging comprehensiveness, and reduces the requirements on the posture of the target object. In the case that there are a plurality of radiation sources, multi-radiation-source tomography is realized by adjusting the angles between the central axes of the beams of the plurality of radiation sources, and the mounting positions, the arrangement directions, and the excitation sequence of the plurality of radiation sources. Tomography is used for imaging the target volume of the target object from different angles in some embodiments, so as to improve the accuracy and detail display of a three-dimensional image. Further, in the process of treatment, the detector and the radiation sources are able to cooperate with each other, i.e., the angles between the central axes of the plurality of radiation sources, and the mounting positions and the arrangement directions of the plurality of radiation sources are adjusted in real time according to a change in the straight-line distance between the detector and the radiation sources. In this way, the position of the target volume of the target object is monitored in real time from multiple angles to ensure the accuracy of treatment.

According to some embodiments of the present disclosure, the present disclosure further provides a radiotherapy system, including: the imaging system as described above; and a radiotherapy device.

FIG. 2 is a schematic diagram of a scenario of a radiotherapy system according to some embodiments of the present disclosure. The radiotherapy system according to the present disclosure is described hereafter with reference to FIG. 2.

In some embodiments, an overlapping volume between a beam 201 emitted by a radiation source 2 of the imaging system and a target volume of a target object 1 always partially or entirely overlaps with a treatment volume of a radiotherapy device 6.

In some embodiments, during movement of a diagnosis and treatment couch 5 from a positioning reference point to a radiotherapy isocenter 601 of the radiotherapy device 6, the imaging system monitors a position of the target volume of the target object 1, and an imaging point 4 of the imaging system moves along a connecting line between the positioning reference point to the radiotherapy isocenter 601;

wherein the connecting line between the positioning reference point to the radiotherapy isocenter 601 is parallel to an X axis, a Y axis, or a Z axis in an IEC coordinate system of the radiotherapy device 6.

It should be noted that during movement of the diagnosis and treatment couch 5, the imaging system monitoring the position of the target volume of the target object 1 means that the imaging system images a part, where the target volume is located, of the target object 1 for many times at a specified frequency. Each imaging has a corresponding imaging point 4, and during movement of the diagnosis and treatment couch 5, the imaging point 4 moves along a connecting line between the positioning reference point to the radiotherapy isocenter 601.

In the IEC coordinate system, the X axis is parallel to a width direction of the diagnosis and treatment couch 5, the Y axis is parallel to a length direction of the diagnosis and treatment couch 5, and the Z axis is parallel to a height direction of the diagnosis and treatment couch 5.

In some embodiments, in the case that the radiotherapy device 6 is a gamma knife device, the radiotherapy isocenter 601 of the radiotherapy device 6 coincides with a nuclear physics central point, and the imaging point 4 of the imaging system moves along a connecting line between the positioning reference point to the nuclear physics central point. The nuclear physics central point refers to a central point of a focus region of rays of the radiotherapy device 6 in the process of treatment.

In the present disclosure, the radiotherapy device 6 is a conventional device in the art that is capable of being equipped with the aforementioned imaging system, and the specific type of the radiotherapy device is not repeated herein.

In some embodiments, the radiation source 2 in the imaging system is mounted on a fixed part or a rotating part of the radiotherapy device 6. In some embodiments, the radiation source 2 is mounted on a fixed part of the radiotherapy device 6. In some embodiments, the radiation source 2 is mounted on a gantry of the radiotherapy device 6 or a fixed part of a device frame of the radiotherapy device 6.

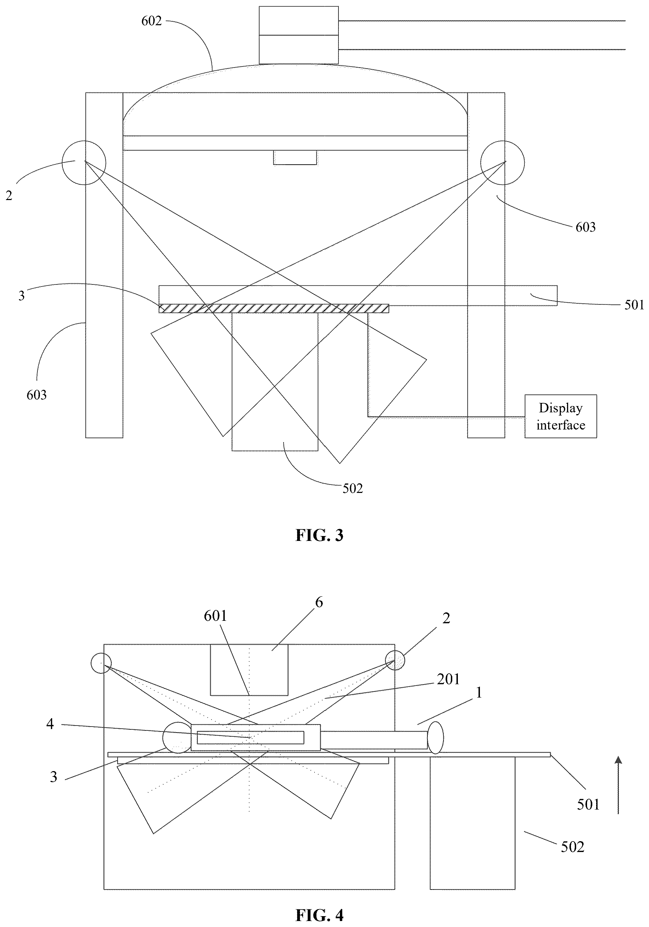

FIG. 3 is a schematic structural diagram of a radiotherapy system according to some embodiments of the present disclosure. Referring to FIG. 3, the radiotherapy system includes an imaging system and a radiotherapy device 6.

The radiotherapy device 6 includes a gantry 603 and a radiotherapy host 602 disposed on the gantry 603.

In some embodiments, the gantry 603 has a frame structure, and the radiotherapy host 602 is supported and fixed by the gantry 603.

In some embodiments, a radiation source 2 of the imaging system is fixed on the gantry 603, and a plurality of radiation sources 2 are arranged in a cranio-caudal direction of a target object 1.

In some embodiments, the diagnosis and treatment couch 5 includes a couch board 501 and a couch base 502, the couch board 501 is fixed on the couch base 502, and the position of the detector 3 is fixed relative to the position of the couch board 501. In some embodiments, the detector 3 is disposed on the couch board 501. In some embodiments, the detector 3 is fixed on the couch board 501.

In some embodiments, the detector of the imaging system is a flat panel detector.

In some embodiments, the couch board 501 of the diagnosis and treatment couch 5 and the detector 3 are capable of reciprocating separately along an X axis, a Y axis, and a Z axis in an IEC coordinate system.

In some embodiments, during movement of the diagnosis and treatment couch 5 from a positioning reference point to a radiotherapy isocenter 601 of the radiotherapy device 6, the imaging system monitors a position of the target volume of the target object 1, and an imaging point 4 of the imaging system moves along a connecting line between the positioning reference point to the radiotherapy isocenter 601;

wherein the connecting line between the positioning reference point to the radiotherapy isocenter 601 is parallel to an X axis, a Y axis, or a Z axis in an IEC coordinate system of the radiotherapy device 6.

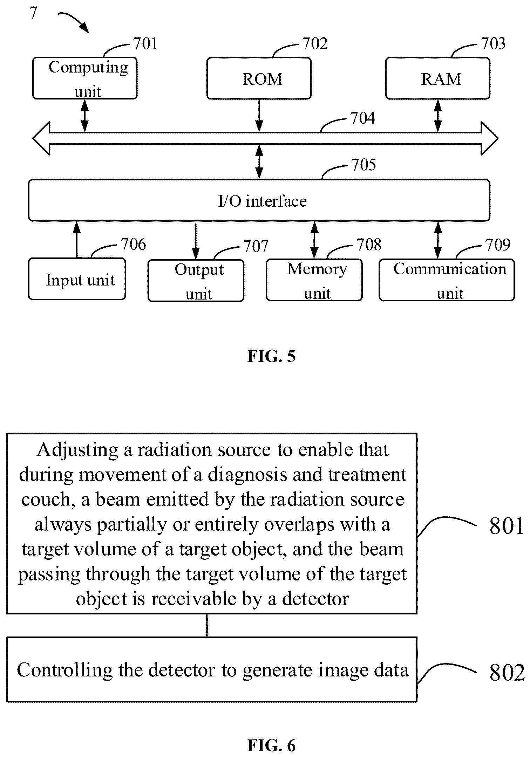

According to some embodiments of the present disclosure, the present disclosure further provides an imaging method applicable to the aforementioned imaging system. Referring to FIG. 6, the method includes the following processes.

In process 801, a radiation source 2 is adjusted, to enable that, during movement of a diagnosis and treatment couch 5, a beam 201 emitted by the radiation source 2 always partially or entirely overlaps with a target volume of a target object 1, and the beam 201 passing through the target volume of the target object 1 is receivable by the detector 3.

In process 802, the detector 3 is controlled to generate image data.

The imaging method provided by the embodiments of the present disclosure is applicable to the imaging system shown in FIG. 1 and the radiotherapy system shown in FIGS. 2 and 3. The imaging method provided by the embodiments of the present disclosure is illustrated hereafter based on the imaging system shown in FIG. 1 and the radiotherapy system shown in FIGS. 2 and 3.

In some embodiments of the present disclosure, a straight-line distance between the flat panel detector 3 and the radiation source 2 is adjusted by the reciprocating motion of the diagnosis and treatment couch 5.

In some embodiments, adjusting the radiation source 2 includes at least one of: adjusting the number of the radiation sources 2; adjusting mounting positions of the radiation sources 2; adjusting arrangement directions of the radiation sources 2; adjusting an excitation sequence of the radiation sources 2; or adjusting angles between central axes of beams 201 of the plurality of radiation sources 2. Because the positions of the diagnosis and treatment couch 5 and the detector 3 are fixed relative to each other, adjusting the position of the diagnosis and treatment couch 5 helps enable that during movement of the diagnosis and treatment couch 5, the beam 201 emitted by the radiation source 2 always partially or entirely overlaps with the target volume of the target object 1, the beam 201 passing through the target volume of the target object 1 is receivable by the detector 3, and the detector 3 generates image data. In this case, a three-dimensional image of the target volume is acquired by reconstructing the acquired image data.

In some embodiments, the imaging method according to the present disclosure is applicable to a radiotherapy system for real-time monitoring of the position of the target volume of the target object 1 during radiotherapy. FIG. 4 is a schematic diagram of a scenario of real-time imaging according to some embodiments of the present disclosure. With reference to FIG. 4, the imaging method includes the following.

The couch board 501 of the diagnosis and treatment couch 5 bears and drives the target object 1 to move upward, until a part, where the target volume is located, of the target object 1 is partially or entirely overlaps with a treatment volume of the radiotherapy device 6; and during movement of the couch board 501, the radiation source 2 is synchronously adjusted to enable that the beam 201 emitted by the radiation source 2 always partially or entirely overlaps with the target volume of the target object 1 during movement of the diagnosis and treatment couch 5.

In some embodiments, an imaging point 4 of the imaging system overlaps with the center of the target volume of the target object 1.

In some embodiments, during movement of the diagnosis and treatment couch 5 from a positioning reference point to a radiotherapy isocenter 601 of the radiotherapy device 6, the imaging system monitors a position of the target volume of the target object 1, and the imaging point 4 of the imaging system moves along a connecting line between the positioning reference point and the radiotherapy isocenter 601;

wherein the connecting line between the positioning reference point and the radiotherapy isocenter 601 is parallel to an X axis, a Y axis, or a Z axis in an IEC coordinate system of the radiotherapy device 6.

In the above embodiments, multi-angle image data are collected by providing the plurality of radiation sources 2, and at the same time, through the cooperation between the detector 3 and the radiation sources 2, i.e., by adjusting, based on a change in the height of the detector 3, the angles between the central axes of the beams 201 of the different radiation sources 2, and the mounting positions and an arrangement sequence of the radiation sources 2 in real time, the position of the target volume of the target object 1 is monitored, so as to ensure the accuracy of treatment.

Further, in some embodiments, multi-directional (multi-angle) image data is acquired by sequentially or simultaneously exciting the radiation sources 2 in different directions (at different angles), and a tomographic image is acquired through tomographic reconstruction. Tomography is capable of imaging in vivo tissues of the target object 1 from different levels so as to improve the accuracy and detail display of the three-dimensional image.

In some embodiments, prior to reconstructing the image data acquired from the detector 3, the imaging method according to the present disclosure further includes preprocessing (such as image correction) the image data to acquire denoised image data.

In some embodiments, the imaging method according to the present disclosure is applicable to a radiotherapy system to position the target object 1. The imaging method includes: adjusting the position of the target object 1 to enable that the beam 201 emitted by the radiation source 2 partially or entirely overlaps with the target volume of the target object 1; exciting the radiation source 2 to emit the beam 201; and controlling the detector 3 to detect the beam passing through the target volume of the target object 1 and generate image data.

According to some embodiments of the present disclosure, the present disclosure further provides an electronic device comprising one or more processors and a memory in communication with the one or more processors, wherein the memory stores one or more instructions executable by the one or more processors, and the one or more instructions, when executed by the one or more processors, cause the one or more processors to perform the imaging method provided by the present disclosure.

FIG. 5 illustrates a schematic block diagram of an exemplary electronic device 7 that is used to implement embodiments of the present disclosure. The electronic device is intended to represent various forms of digital computers, such as, laptop computers, desktop computers, workstations, personal digital assistants, servers, blade servers, mainframe computers, and other suitable computers. In some embodiments, the electronic device also represents various forms of mobile devices, such as, personal digital assistants, cellular phones, smartphones, wearable devices, and other similar computing devices. The components, their connections and relationships, and their functions shown herein are provided merely as examples and are not intended to limit the embodiments of the present disclosure described and/or claimed herein. In some embodiments, the electronic device is the imaging control processing device described above.

As shown in FIG. 5, the electronic device 7 includes a computing unit 701. The computing unit 701 is capable of performing various appropriate actions and processing by executing computer programs stored in the read-only memory (ROM) 702 or computer programs loaded from the storage unit 708 into the random-access memory (RAM) 703. In some embodiments, the RAM 703 further stores various programs and data required for the operation of the electronic device 7. The computing unit 701, ROM 702, and RAM 703 are interconnected via a bus 704. An input/output (I/O) interface 705 is also connected to the bus 704.

Multiple components within the electronic device 7 are connected to the I/O interface 705, including: an input unit 706, such as, keyboards, mice, etc.; an output unit 707, such as, various types of displays, speakers, etc.; a storage unit 708, such as, disks, optical discs, etc.; and a communication unit 709, such as, network cards, modems, wireless communication transceivers, etc. The communication unit 709 enables the electronic device 7 to exchange information/data with other devices via computer networks (such as the Internet) and/or various telecommunication networks.

In some embodiments, the computing unit 701 includes various general-purpose and/or specialized processing components having processing and computation functions. Examples of the computing unit 701 include, but are not limited to, central processing units, graphics processing units (GPUs), various specialized artificial intelligence (AI) computing chips, various computing units running machine learning model algorithms, digital signal processors, and any suitable processors, controllers, microcontrollers, etc. The computing unit 701 performs the various methods and processes described above, such as the imaging method. For example, in some embodiments, the imaging method is implemented as a computer software program tangibly embodied on a machine-readable medium, such as the storage unit 708.

According to embodiments of the present disclosure, the present disclosure further provides a non-transitory computer-readable storage medium storing one or more computer instructions, wherein the one or more computer instructions, when executed by a processor of an electronic device, cause the electronic device to perform the imaging method provided by the present disclosure.

According to embodiments of the present disclosure, the present disclosure further provides a computer program product comprising a computer program, wherein the computer program, when executed by a processor, causes the processor to perform the imaging method provided by the present disclosure.

In some embodiments, a part or all of the computer program is loaded and/or installed onto the electronic device 7 via the ROM 702 and/or the communication unit 709. The computer program, when loaded into the RAM 703 and executed by the computing unit 701, causes the computer unit 701 to perform one or more processes of the imaging method described above. Alternatively, in other embodiments, the computing unit 701 is configured to perform the aforementioned imaging method by any other suitable means (e.g., via firmware).

In some embodiments, various embodiments of the systems and technologies described herein are implemented in digital electronic circuit systems, integrated circuit systems, field programmable gate arrays, application specific integrated circuits, application specific standard parts (ASSPs), systems on chip (SOCs), complex programmable logic devices (CPLDs), computer hardware, firmware, software, and/or combinations thereof. These various implementations include: implementations through one or more computer programs, wherein the one or more computer programs are executable and/or runnable on a programmable system including one or more programmable processors; wherein the one or more programmable processors are dedicated or general-purpose programmable processors, capable of receiving data and instructions from a storage system, one or more input devices, and one or more output devices, and transmitting data and instructions to the storage system, the one or more input devices, and the one or more output devices.

In some embodiments, the one or more program codes for implementing the methods of the present disclosure are written in any combination of one or more programming languages. In some embodiments, the one or more program codes are provided to a processor or controller of a general-purpose computer, a dedicated computer, or other programmable data processing apparatus, wherein the one or more program codes, when executed by the processor or controller, cause the processor or controller to perform the functions/operations specified in the flowchart and/or block diagram. In some embodiments, the one or more program codes are executed entirely on a machine, partially on a machine, partially on a machine and partially on a remote machine as a standalone software package, or entirely on a remote machine or server.

In the context of the present disclosure, in some embodiments, a machine-readable medium is a tangible medium that contains or stores a program that is used by or used in conjunction with an instruction-executing system, apparatus, or device. In some embodiments, the machine-readable media include machine-readable signal media or machine-readable storage media. In some embodiments, machine-readable media include, but are not limited to, electronic, magnetic, optical, electromagnetic, infrared, or semiconductor systems, apparatuses, or devices, or any suitable combination thereof. More specific examples of machine-readable storage media include electrical connections based on one or more wires, portable computer disks, hard disks, random access memories, read-only memories, erasable programmable read-only memories, optical fibers, portable compact disc read-only memories, optical storage devices, magnetic storage devices, or any suitable combination thereof.

In some embodiments, to achieve interaction with users, the systems and techniques described herein are implemented on a computer that includes: a display device for displaying information to the user, such as, a cathode ray tube (CRT) or a liquid crystal display (LCD) monitor; and a keyboard and pointing device (e.g., a mouse or trackball) through which the user is able to provide input to the computer. In some embodiments, other types of devices are also used to achieve user interaction. In some embodiments, feedback provided to the user includes any form of sensory feedback (e.g., visual, auditory, or tactile feedback); and the input from the user is received in any form (including acoustic input, voice input, or tactile input).

In some embodiments, the systems and technologies described herein are implemented in computing systems including back-end components (e.g., as data servers), or computing systems including middleware components (e.g., application servers), or computing systems including front-end components (e.g., user computers with graphical user interfaces or web browsers, through which the user is able to interact with the system and technology implementations described herein), or a computing system including any combination of such back-end components, middleware components, or front-end components. The components of the system are interconnected via digital data communication in any form or medium (e.g., a communication network). Examples of the communication network include: local area networks (LANs), wide area networks (WANs), and the Internet.

In some embodiments, a computer system includes a client and a server. The client and server are typically geographically separated and interact primarily through communication networks. The client-server relationship is established by computer programs running on corresponding computers that maintain a client-server relationship with each other. In some embodiments, the server is a cloud server, a server within a distributed system, or a server incorporating blockchain technology.

It should be understood that the various forms of the processes shown above are used by reordering in some embodiments, or some processes are added or deleted in some embodiments. In some embodiments, the processes described in the present disclosure are executed in parallel, sequentially, or in different orders, which is not limited in the present disclosure, as long as the desired results of the technical solutions disclosed herein are achieved.

The specific embodiments described above do not constitute limitations on the scope of protection of the present disclosure. Those skilled in the art will appreciate that various modifications, combinations, sub-combinations, and substitutions may be made based on design requirements and other factors. Any modifications, equivalent substitutions, and improvements made within the concept and principles of the present disclosure should be included within the scope of protection of the present disclosure.

## Claims

1. An imaging system, configured to image a target object (1), comprising:
a radiation source (2), configured to emit a beam (201) to the target object (1);
a detector (3), arranged opposite to the radiation source (2); and
a diagnosis and treatment couch (5), disposed between the radiation source (2) and the detector (3), fixed relative to the detector (3), and configured to bear and drive the target object (1) to move;
wherein during movement of the diagnosis and treatment couch (5), the beam (201) emitted by the radiation source (2) always partially or entirely overlaps with a target volume of the target object (1), and the beam (201) passing through the target volume of the target object (1) is receivable by the detector (3).

2. The imaging system according to claim 1, wherein a plurality of radiation sources (2) are provided.

3. The imaging system according to claim 2, wherein central axes of beams (201) of the plurality of radiation sources (2) intersect at an imaging point (4).

4. The imaging system according to claim 2, wherein the imaging system satisfies at least one of:
angles between central axes of beams (201) of the plurality of radiation sources (2), and mounting positions, arrangement directions, and an excitation sequence of the plurality of radiation sources (2) are all adjustable; or
the plurality of radiation sources (2) are arranged in a cranio-caudal direction of the target object (1).

5. The imaging system according to claim 1, wherein a straight-line distance between the detector (3) and the radiation source (2) is adjustable.

6. The imaging system according to claim 1, wherein the diagnosis and treatment couch (5) comprises a couch board (501) and a couch base (502), the couch board (501) being fixed on the couch base (502), wherein a position of the couch board (501) is fixed relative to a position of the detector (3).

7. The imaging system according to claim 6, wherein the imaging system satisfies at least one of:
the detector (3) is disposed on the couch board (501); or
the couch board (501) and the detector (3) are capable of reciprocating separately along an X axis, a Y axis, and a Z axis in an IEC coordinate system.

8. The imaging system according to claim 1, comprising:
an imaging control processing device, capable of controlling, based on an imaging mode selected by a user, the imaging system as defined in any one of claims 1 to 7 to perform imaging;
wherein the imaging mode comprises tomography, KV imaging, or CBCT imaging.

9. The imaging system according to claim 1, wherein the detector (3) is sized to be able to cover a part, wherein the target volume is located, of the target object (1).

10. A radiotherapy system, comprising: the imaging system as defined in any one of claims 1 to 9 and a radiotherapy device (6).

11. The radiotherapy system according to claim 10, wherein an overlapping volume between the beam (201) emitted by the radiation source (2) and the target volume of the target object (1) always partially or entirely overlaps with a treatment volume of the radiotherapy device (6).

12. The radiotherapy system according to claim 10, wherein during movement of the diagnosis and treatment couch (5) from a positioning reference point to a radiotherapy isocenter of the radiotherapy device (6), the imaging system monitors a position of the target volume of the target object (1), and an imaging point (4) of the imaging system moves along a connecting line between the positioning reference point and the radiotherapy isocenter;
wherein the connecting line between the positioning reference point and the radiotherapy isocenter is parallel to an X axis, a Y axis, or a Z axis in an IEC coordinate system of the radiotherapy device (6).

13. An imaging method, applicable to the imaging system as defined in any one of claims 1 to 9 or the radiotherapy system as defined in any one of claims 10 to 12,
wherein the method comprises:
adjusting (801) the radiation source, to enable that, during the movement of the diagnosis and treatment couch, the beam emitted by the radiation source always partially or entirely overlaps with the target volume of the target object, and the beam passing through the target volume of the target object is receivable by the detector; and
controlling (802) the detector to generate image data.

14. The imaging method according to claim 13, wherein a plurality of radiation sources are provided, and adjusting (801) the radiation source comprises at least one of:
adjusting a number of the plurality of radiation sources;
adjusting mounting positions of the plurality of radiation sources;
adjusting arrangement directions of the plurality of radiation sources;
adjusting an excitation sequence of the plurality of radiation sources; or
adjusting angles between central axes of beams of the plurality of radiation sources.

15. An electronic device, comprising:
a processor; and
a memory, configured to store one or more instructions executable by the processor;
wherein the processor, when executing the one or more instructions, is caused to perform the imaging method as defined in claim 13 or 14.
